(19) **Europäisches Patentamt**
**European Patent Office**
**Office européen des brevets**

(11) **EP 1 588 161 B1**

(12) **EUROPEAN PATENT SPECIFICATION**

(45) Date of publication and mention
of the grant of the patent:
**24.10.2007 Bulletin 2007/43**

(51) Int Cl.:
***G01N 33/49*** *(2006.01)*   ***G01N 33/86*** *(2006.01)*

(21) Application number: **04704993.7**

(86) International application number:
**PCT/EP2004/000595**

(22) Date of filing: **24.01.2004**

(87) International publication number:
**WO 2004/068138 (12.08.2004 Gazette 2004/33)**

(54) **SYSTEM AND METHOD FOR MEASURING COAGULATION TIME WITHOUT THERMOSTATIC CONTROL**

SYSTEM UND VERFAHREN ZUR MESSUNG VONKOAGULATIONSZEIT OHNE THERMOSTATISCHE STEUERUNG

SYSTEME ET PROCEDE POUR MESURER UN TEMPS DE COAGULATION SANS COMMANDE THERMOSTATIQUE

(84) Designated Contracting States:
**AT BE BG CH CY CZ DE DK EE ES FI FR GB GR HU IE IT LI LU MC NL PT RO SE SI SK TR**

(30) Priority: **01.02.2003 EP 03002254**

(43) Date of publication of application:
**26.10.2005 Bulletin 2005/43**

(73) Proprietors:
• **Roche Diagnostics GmbH**
**68305 Mannheim (DE)**
Designated Contracting States:
**DE**
• **F. Hoffmann-Roche AG**
**CH-4070 Basel (CH)**
Designated Contracting States:
**AT BE BG CH CY CZ DK EE ES FI FR GB GR HU IE IT LI LU MC NL PT RO SE SI SK TR**

(72) Inventors:
• **HILL, James, L.**
**82340 Feldafing (DE)**
• **UNKRIG, Volker**
**68526 Ladenburg (DE)**
• **RIEGER, Ewald**
**67240 Bobenheim-Roxheim (DE)**

(74) Representative: **Pfeifer, Hans-Peter et al**
**Durm & Partner**
**Patentanwälte**
**Beiertheimer Allee 19**
**76137 Karlsruhe (DE)**

(56) References cited:
**US-A- 5 580 744      US-A- 5 789 644**
**US-A- 6 066 504      US-A- 6 150 174**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

**Description**

Many medical problems relate to the coagulation of blood. In particular during treatment with anticoagulant medication, a patient's coagulation parameters fluctuate continuously. Such fluctuations can cause severe problems. For example if a patient is treated with an anticoagulant drug such as Heparin or Marcumar, it is important that his or her coagulation parameters remain within a defined range of values to avoid complications. Only in this way it is possible to reduce effectively the number of blood clots while simultaneously avoiding bleeding complications. A rapid, precise method for the continuous monitoring of blood coagulation parameters that meets all therapeutic needs is therefore required.

Currently, in particular three coagulation parameters are of medical interest, namely prothrombin time (PT), activated partial thromboplastin time (APTT) and activated clotting time (ACT). PT mainly serves for monitoring the effect of vitamin K antagonists on coagulation (which influence factors II, V, VII and X of the coagulation cascade). The PT test measures the activation of the extrinsic pathway by addition of tissue thromboplastin.

APTT is used primarily to monitor heparin therapy. The test detects factor changes in the intrinsic coagulation cascade (factors VIII, IX, XI, XII and other enzymes and factors). The test reagents for this type of test have not yet been standardized and therefore differences in the heparin sensitivity of reagents from different manufacturers are considerable.

ACT is determined to monitor heparinization in situations where an APTT test cannot be performed, because the patient was administered a high dose of heparin.

Traditionally, the coagulation parameters are determined by "wet chemistry" tests. An aliquot of blood sample is mixed with liquid reagents and the point of time at which the blood clots is detected. The results are indicated either directly (in seconds) or in the form of derived quantities such as ratio to a respective normal value (in percent). With respect to PT further common quantities for indication of the test results are % Quick and INR (International Normalized Ratio).

Since several years so-called "dry chemistry" tests of coagulation parameters have become available. They are performed by means of test systems comprising disposable reagent carrier elements (often designated "test elements") and an evaluation instrument which generally is adapted for the evaluation of a particular type of test element from a particular manufacturer. The test element contains the reagent system necessary for the particular test and, preferably, suitable information for the evaluation of the test such as the test type, the lot number and the expiration date.

The invention refers to such dry chemistry tests. Each test element is designed to allow a drop of a blood or plasma sample (which depending on the test may be pretreated, in particular by reaction with a preparatory anticoagulant) applied thereto to contact and dissolve a reagent system (which normally comprises a plurality of reagents) present in the test element and suitable to initiate the reaction sequence of the blood coagulation path. After mixing of the sample with the reagents the resulting coagulation detection liquid is present in a coagulation detection zone of the test element. The coagulation process is monitored by observing a measurable property of the coagulation detection liquid which changes in a characteristic manner when a defined reaction step of the coagulation path occurs. The system comprises means for detecting such change and generating a corresponding signal by the measurement and evaluation electronics of the instrument. The measurement and evaluation electronics also includes a time measurement electronics for measuring a coagulation time required for - the change to occur. This time is converted into the desired coagulation parameter (in the appropriate units). To this end evaluation data stored in the instrument and/or in the test element can be used. The results are then displayed on a display of the instrument and/or forwarded to further evaluation, for example by a separate computer system.

Such a system is commercially available from the applicants under the trade name CoaguChek®. Further details may be found in the appropriate literature including US patent 5,789,664 and WO 01/11356. The sample may be whole blood or plasma. Hereafter reference is made to blood as an example. This should, however, not be understood as a limitation to the general applicability of the invention.

Known coagulation test systems differ inter alia with respect to the measurable property of the coagulation detection liquid which is used to measure the coagulation time and with respect to the arrangement by which the contacting of the sample with the reagents is achieved as well as by the design of the detection zone:

- In some tests the measurable property of the liquid is its viscosity which can be detected e.g. by including magnetic particles into the reagent system. The moveability of these particles can be detected by means of an alternating magnetic field. In such systems the increase of the viscosity caused by the onset of coagulation is the change marking the end of the coagulation time.

- Alternatively a chemical constituent, the concentration of which changes at a defined point of the coagulation path, can be used to mark the end of the coagulation time. In particular the enzyme thrombin, the final protease for both plasmatic coagulation pathways, can be monitored by known means, including a reagent present in the reagent system of the test element which is suitable to generate an electrical or optical signal which can be measured by the evaluation instrument (see e.g. WO 01/63271).

- With respect to the physical arrangement of the test element it has been proposed to use a single permeable porous membrane which is fastened to a plastic handle similar as with traditional test strips (US patent 5,580,744). In such test elements the sample is directly applied to the membrane in which the reagent system is contained.

- In an alternative arrangement the sample application point of the test element is located at a distance from the coagulation detection zone and the transport of the sample liquid from the former to the latter is accomplished by a capillary channel (see US patent 5,789,664).

While tests for coagulation parameters have become relatively simple, in particular due to the introduction of dry chemistry tests, it remains an important task to further simplify the design and thus reduce the cost without sacrificing accuracy. For example, if a person received a replacement cardiac valve his or her long-term health highly depends on the fact that the coagulation status remains reliably between certain boundaries. To this end inexpensive small battery-operated instruments should be available to be used by the patients themselves in order to monitor their blood coagulation status. Similar requirements apply to "point of care (POC)" testing by the medical profession.

Therefore the invention addresses the task to simplify the design of systems for determining coagulation parameters while simultaneously maintaining the required degree of accuracy.

This task is achieved by a system for the determination of a coagulation parameter of blood or plasma samples of patients, said system comprising a disposable test element including a reagent system to be mixed with the sample, thereby forming a coagulation detection liquid, and a coagulation detection zone where the coagulation detection liquid is contained for monitoring a measurable property of the coagulation detection liquid, said measurable property changing during coagulation, and an instrument with a holder for holding said disposable test element inserted therein and with a measurement and evaluation electronics adapted for detecting a signal which corresponds to the measurable property of the coagulation detection liquid and including a time nieasurement_electronics for measuring a coagulation time required for said change to occur for deriving the desired coagulation parameter therefrom, wherein the coagulation time is determined at a non-standard temperature of the coagulation detection liquid different from a standard temperature for the respective coagulation parameter, said non-standard temperature being measured by a temperature measurement device, the instrument has a non-volatile memory containing data which define a mathematical relationship of coagulation time versus temperature which relationship is independent of the individual patient whose blood is examined, and the coagulation parameter for the standard temperature is calculated from the coagulation time measured at said non-standard temperature, using said mathematical relationship.

Known systems for the determination of coagulation parameters generally comprise some kind of thermostating device designed to maintain during the coagulation measurement a defined standard temperature, usually 37°C. This requires an electric heater and an electronic temperature control system, Based on the invention such a thermostating device is not necessary. Rather the coagulation measurement can be made at a convenient temperature (e.g. room temperature). Nevertheless accurate values of the desired coagulation parameter can be determined which are directly comparable to those determined at the standard temperature. This leads to a plurality of advantages:

- For small portable battery operated coagulation instruments the heating system is expensive and consumes more battery energy than all other components combined. The volume, weight and cost of the instruments can be reduced substantially if no heating system and a smaller battery are required.

- In order to secure an exact control of the desired standard temperature test elements of known dry chemistry systems often have a long capillary for guiding the sample liquid from the point of sample application (outside the instrument) into the interior of the instrument where the thermostating device is located. If no such long capillary is necessary the sample volume can be reduced.

- The warm up time required with known instruments before testing is eliminated or reduced. This simplifies the use of the system, in particular in home monitoring or POC applications.

- For INR calibration a manual technique is generally used which causes difficulties in controlling the standard (37°C) temperature. Errors due to temperature deviations can be reduced if the technique can be performed at room temperature.

During the experimental work on which the invention is based it has been determined that the change of coagulation time parameters versus temperature can be described by a functional relationship which is specific for a particular instrument and a particular reagent system but surprisingly is independent on the sample. Therefore the same functional relationship f(T) (which may e.g. be a linear function or a polynomial) describes the temperature dependence no matter whose blood is being examined. Taking PT as an example this may be expressed mathematically:

$$(1) \quad PT^{37} = f(T) * PT^{T}$$

where

PT$^{37}$: PT value for the standard temperature of 37°C

PT$^{T}$: PT value for a (lower) non-standard temperature.

Generally in the field of clinical chemistry it is not unusual to perform a temperature correction calculation if a test is temperature dependent and the actual test temperature differs from a desired standard test temperature. For example tests for determining the concentration of glucose in blood are temperature dependent and it has been proposed to eliminate errors caused by temperature variations by an appropriate correction calculation (see e.g. US patents 5,405,511 and 5,972,715).

Coagulation detection is, however, fundamentally different from the detection of the concentration of an analyte such as glucose. The temperature dependence of glucose tests is caused by influencing factors which are independent of the individual sample which is tested. In particular the enzymatic reaction on which the test is based depends on the temperature. A coagulation test is fundamentally different since it is essentially an experimental model of the natural coagulation process which - as is well known - involves a highly complicated reaction of more than ten factors and enzymes in the blood of the particular patient. The fact that the coagulation behaviour of blood is different for every individual is also apparent from the fact that the dosage: of corresponding medication (e.g. Heparin or Marcumar) has to be individually adapted.

Therefore in coagulation testing the quantity which is measured depends on the particular individual sample, i.e. the individual whose blood is tested and the status of his or her coagulation system. Regarding the temperature dependence the same had to be assumed. Therefore it could not be expected that, by examining the blood of a limited number of patients at different temperatures with the same test system (same type of instrument and same reagent system, preferably of the same manufacturing lot) a universal f(T)-curve could be generated which allows calculation of PT$^{37}$ from PT$^{T}$ values determined at a non-standard temperature for additional patients with quite different blood.

In the prior art PT and APTT tests have generally not been performed at temperatures outside the standard range at about 37°C. An exception is described in

A. Uldall: "Prothrombin time standardization and temperature problems", Clinica Chimica Acta, 1980, 39-44.

This publication refers to PT tests performed in a glass tube which are immersed into a water bath to allow exact control of the temperature. The author refers to problems caused by deviations from the standard temperature of 37°C, e.g. in case the glass tube is not immersed sufficiently deeply and by the fact that the tube has to be withdrawn from the water bath repeatedly in order to check the coagulation status. In order to reduce these problems a lowering of the generally accepted standard temperature from 37°C to 30°C is considered.

US-patent 5,031,619 refers to a Bleeding Time Test. In such tests an incision of predetermined dimensions is applied to the skin of a patient and the time is measured from a moment that bleeding through the incision begins to the moment coagulation appears to occur. Such bleeding time measurements are used as screening tests for evaluation of the hemostatic adequacy of platelets which depends on platelet number and platelet function. It is a qualitative (or at most semi-quantitative) test allowing screening for certain disorders (e.g. Glanzmann's thrombasthenia). The patent refers to the earlier known fact that the rate at which external bleeding occurs is roughly inversely proportional to the temperature of the subject's skin and suggests to use a fixed factor of 0.05 per degree in order to compensate for variations of the temperature of the subject.

Above identified US patent 5,580,744 describes a large number of experiments all of which are performed at a constant standard temperature. As a theoretical alternative the possibility is mentioned to perform coagulation detection reactions at a deviating temperature such as room temperature. An equation for a possible correction calculation is given but the patent contains no explanation how this should be accomplished in practice. Nothing is mentioned about the problems caused by the fact that the coagulation path way is different for each individual. Evidently the author did not address this question in view of the fact that his system is specifically designed to be used by a single person.

The invention will be further described hereafter with reference to the figures wherein

Fig. 1     shows a crossectional view of a coagulation test system with the test element inserted into the evaluation instrument;

Fig. 2     shows a schematic design diagram of a preferred system according to the invention;

Fig. 3     shows an enlarged view of an element temperature sensor shown in fig. 2;

Fig. 4     shows a graphical representation of the ratio PT$^{37}$/ PT$^{T}$ vs. T[°C] /37 for a series of experiments performed in the context of the invention;

Fig. 5     shows a graphical representation of experimental results comparing the invention with a conventional determination of INR values;

Figs. 6 to 10 show results corresponding to figure 4 performed with different coagulation parameter tests.

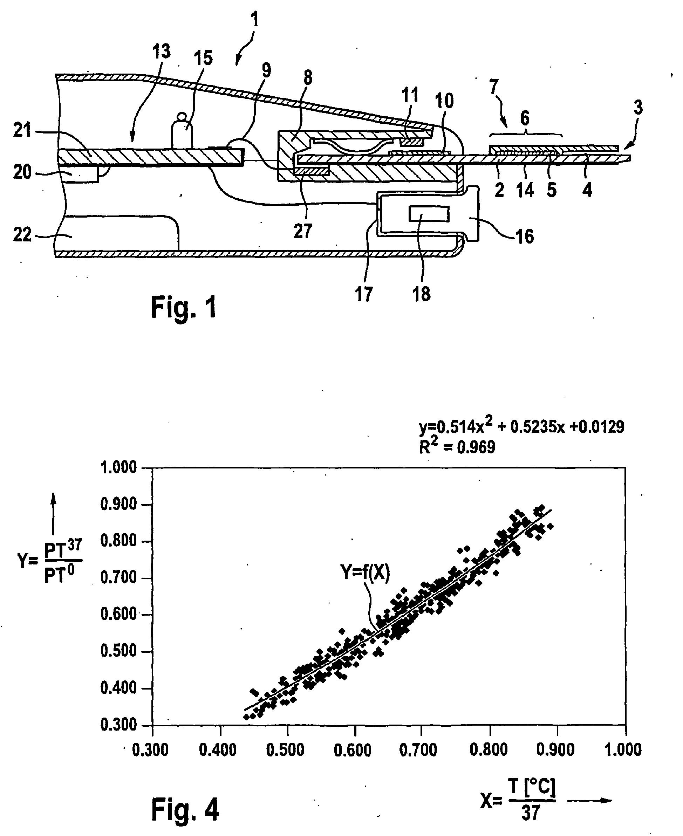

Figure 1 is a crossectional schematic representation of a coagulation test system comprising an evaluation instrument 1 and a disposable test element 2. The test element has a sample application opening 3, a capillary channel 4 and a space 5 which serves as reaction chamber and coagulation detection zone 6. The channel 4 is very short and can even be omitted because with a system according to the invention coagulation detection zone 6 need not be thermostated and is, when test element 2 is inserted in a holder 8 of instrument 1, preferably located outside the housing of the instrument 1 close to sample application opening 3.

In prior art devices easy application of the sample required that the sample application opening is outside of the instrument whereas thermostating required that the coagulation detection zone of the test element is inside the instrument housing. The sample was transported from the former to the latter by a long capillary path. This again required a large sample volume not only because of the length of the path but also because of a sufficiently large capillary path crossection required for adequate speed of liquid transport. In contrast the invention allows a very short transport distance (of preferably less than 1 cm or even less than 0,5 cm) and an extremely small sample volume (preferably less than 5 $\mu$l, most preferably less than 2 $\mu$l).

Space 5 receives the coagulation detection liquid which is formed by mixing of a sample applied to the sample application field 3 and a reagent system (not shown). Coagulation detection is performed in the coagulation detection zone 6 by detecting a change of a coagulation-related measurement quantity of the liquid contained therein. The resulting signals are transmitted via lines 9 to a measurement and evaluation unit 13 which controls the operation of the instrument. Coagulation detection can be performed by any of the methods known from the prior art, in particular by optical or electrochemical means, see e.g. US patent 5,789,664 and WO 01/11356. Details of a preferred embodiment will be described below.

The system of the invention comprises a temperature measuring device 7 which is suitable for measuring the temperature of the coagulation detection zone 6. A temperature measurement sensor, in particular a thermistor, is integrated as element temperature sensor 14 into test element 2 and connected by plug-in contacts to the electronics of the instrument. Additionally a instrument temperature sensor 15 may be provided and used as will be described in more detail below.

Test element 2 carries information relating to test type and reagent lot in an information field 10 which is evaluated by an element information reader 11. The detected information signal is transmitted via lines (not shown) to the measurement and evaluation unit 13. Information field 10 and information reader 11 are shown only schematically. A barcode and an appropriate barcode detector can be used as a preferred example.

A ROM key 16 sits exchangeably in a ROM key holder 17 and is connected to the central measurement and evaluation unit 13 for data exchange therewith. It includes a memory 18 in which data required for evaluation of the test are stored. These data may depend on the manufacturing lot of the test element. Preferably information field 10 contains identification data which are specific for the manufacturing lot of the particular test element 2. This information is read by information reader 11 and compared with lot identification data stored in ROM key 16 in order to make sure that the ROM key inserted into the instrument corresponds to the manufacturing lot of the test element 2.

Memory 18 of ROM key 16 also includes data describing the mathematical relationship of coagulation time versus temperature which is used for calculating the desired coagulation parameter for a standard temperature from the coagulation time measured at a non-standard temperature. Alternatively these data may also be contained in a permanent memory of instrument 1.

The central measurement and evaluation unit 13 may consist of conventional electronic circuitry including an ASIC 20 and a circuit board 21. It comprises a programmable microprocessor for controlling the instrument functions and performing the required calculations. In particular it combines the signal information received from coagulation detection device 6, element information device 11, non-volatile memory 18 and temperature measuring device 7 to derive the desired coagulation parameter. This result is transmitted to a display (not shown) of instrument 1. Power for the instrument operation is provided by a battery 22.

Most parts of the system shown in figure 1 are conventional and no further details need to be described. Deviating from the prior art, however, instrument 1 must not be equipped with a thermostating system including a heater and electric heating control. Rather it uses a temperature measuring device 7 for measuring (directly or indirectly) the temperature of the coagulation detection liquid in the coagulation detection area and the mathematical relationship stored in memory 18 to calculate the coagulation parameter for the standard temperature.

During experimental evaluation of the invention it has been adequately demonstrated that measuring the temperature adjacent to the space in which the coagulation detection liquid is contained is sufficient to allow accurate calculation of a desired standard coagulation parameter (which would have been obtained at a controlled temperature of approximately 37°C) from measurements performed at a deviating (generally substantially lower) temperature using a mathematical relationship which is independent of the-respective sample and can therefore be stored in a non-volatile memory of the instrument and repeatedly used for a plurality of different individuals (patients). The measurement and evaluation unit 13 is adapted to perform the required calculations.

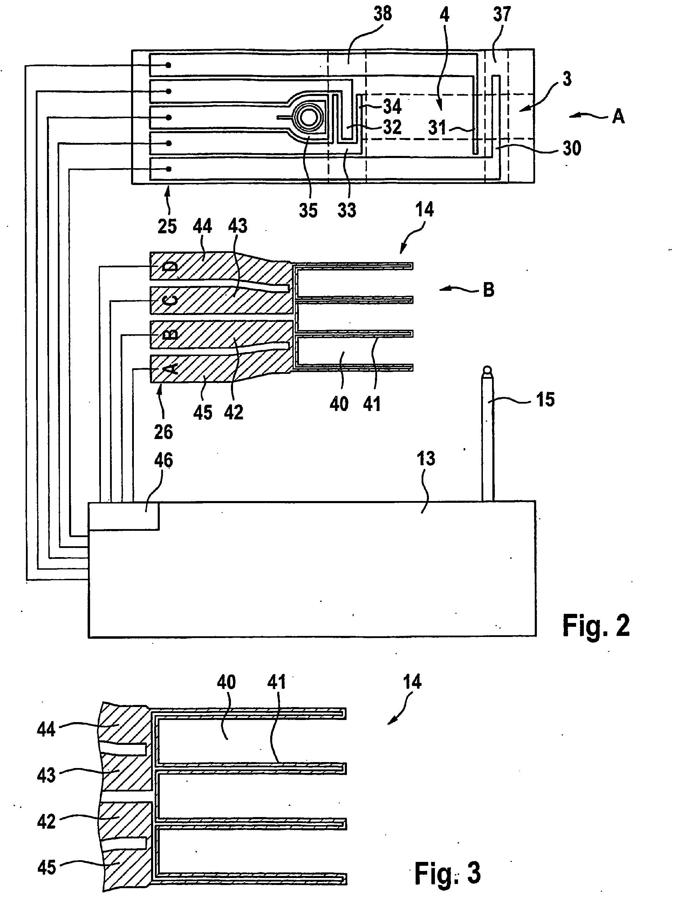

Figures 2 and 3 show important design features of a preferred embodiment of a coagulation test system partly as top view showing a preferred layout of test element layers and partly as block diagram of the electronics. This preferred embodiment is in particular characterized by two aspects:

- Coagulation detection is performed by an arrangement of reagents and electrodes as shown in the upper part of figure 2.

- The required accurate measurement of the temperature of the coagulation detection zone is achieved by the combined use of an element temperature sensor 14 integrated into the test element and an instrument temperature sensor 15 located at the instrument (relatively remote from the coagulation detection zone 6)

Both these aspects can be combined. In this case the test element preferably has two layers of electric leads such as layers A and B shown in figure 2. Such layers can be applied in known manner onto a non-conducting (plastic) carrier foil and separated by an isolating layer. Electrical contact between the electrode arrangement provided by the leads of the layers and the instrument is achieved by element contact pads 25 and 26 respectively which provide electrical connection with corresponding instrument contacts 27 (figure 1). In order to provide transport of the sample liquid from a sample application opening 3 to a coagulation detection zone 6 a capillary channel 4 is provided on top of layer A. All these design elements can be embodied by known means therefore no further description thereof is required.

Electrode arrangement A of figure 2 comprises five electrodes including a first counter electrode 30, a drop detection electrode 31, a working electrode 32, a second counter electrode 33 with two fingers 34 surrounding the working electrode 32 and a stirrup-shaped fill detection electrode 35. The electrode structure may be produces e.g. by laser ablation techniques out of gold layers of about 50 $\mu$m thickness.

Counter electrode 30 is located close to fill opening 3 and is covered by a reference reagent layer 37 containing e.g. Ag/AgCl. Working electrode 32 and second counter electrode 2 are covered by a coagulation detection reagent 38 which includes the required reagent system to start the reactions of the coagulation path. Reagent layer 38 also contains any reagents required for detection of the appropriate measurable property, in particular in the preferred case of enzymatic detection of coagulation a substrate of the respective enzyme, for example the substrate electrozyme TH of the enzyme thrombin.

A test protocol performed with the system shown in figure 2 may include the following steps:

- The instrument is switched on automatically by inserting a test element and usual operation checks are performed.

- When a sample drop is applied to sample opening 3 of capillary channel 4 and the liquid bridges first counter electrode 30 and drop detection electrode 31 this may be detected by applying a suitable AC-potential to these electrodes and detecting a sharp drop of the impedance when both electrodes are contacted by the liquid.

- When the blood sample is further drawn into capillary channel 4 (by capillary action) its arrival at working electrode 32 and second counter electrode 34 can again be detected by applying a suitable AC-potential to these electrodes. The wetting of working electrode 32 is also a suitable starting point for the coagulation time measurement as it coincides with dissolving of reagent layer 38 and therefor with the start of the coagulation reaction sequence.

- Complete fill of capillary 4 is detected by a current flowing between fill detection electrode 34 and second counter electrode 33.

- An enzyme activity is detected as measurable property of the coagulation detection liquid by electrochemical means. To this end after complete fill of capillary 4 a suitable constant DC-potential is applied to working electrode 32 with reference to first counter electrode 30. Thereafter current measurements are taken at suitable intervals (e.g. 0.1 seconds). When at the end of the coagulation path the enzyme Thrombin is formed it cleaves an electrochemically active group from the substrate contained in reagent layer 38 thereby causing an increase of the current which is characteristic of the enzyme formation. This change defines the end of the coagulation time which is used for deriving the desired coagulation parameter in known manner.

Layer B shown in figure 2 is an example of electric leads suitable as element temperature sensor 14. Its temperature sensitive range 40 is defined by the area in which a very thin and narrow electric thermistor-conductor runs, typically in a meandering manner as shown in more detail in figure 3. Conductor 41 can be made from typical thermistor material with a large dependence of resistance versus temperature. However, also materials more common for test element manufacturing including a gold layer of sufficiently small thickness (less than 100 $\mu$m) has been found suitable.

The resistance of the thermistor-conductor 41 is measured by a four-terminal arrangement where two terminals 42,43 are used for feeding a constant current into thermistor-conductor 41 and two separate terminals 44,45 are used to measure the resistance in current-free manner.

During operation of the system the temperature signal generated by element temperature sensor 14 is used in combination with the signal generated by instrument sensor 15 to determine a reliable temperature value of high accuracy to be used for calculation of the coagulation parameter for the standard temperature from a measurement performed at a (lower) non-standard temperature:

- The element temperature sensor 14 is only used to provide information about temperature changes in close proximity of the coagulation detection zone 6. It is therefore not necessary for this sensor and the temperature measurement electronics 46 (which is part of measurement and evaluation electronics 13) to allow temperature measurement in absolute quantities.

- Instrument temperature sensor 15 is used to provide temperature information relative to the standard temperature. This temperature information has to be absolute in the sense that the measured difference from the standard temperature is (with a high degree of accuracy) the same for all instruments which is a necessary requirement for using the same mathematical relationship of coagulation time versus temperature for the temperature conversion of all instruments.

In practical use after insertion of a test element 2 into instrument 1 the temperature of element temperature sensor 14 is monitored to derive an information about the change of the element temperature versus time or in other words the speed of element temperature change. Only when this speed of change falls below an acceptable value (in other words only when the temperature of the element 2 is sufficiently constant) a signal is given that a drop of a sample may be applied to the test element to perform a determination of coagulation parameter. If this condition is met the temperature value of instrument temperature sensor 15 is used as "true" temperature for the conversion.

Preferably not only the temperature change of element temperature sensor 14 but also the temperature change of instrument temperature sensor 15 is monitored. Even better results are achieved if a coagulation determination takes place only when the speed of change of both temperature sensors is below a suitable limit (which may be different for both sensors) indicating a highly constant temperature environment.

During experimental evaluation of the invention it has been found that by these means a temperature measurement is possible which fulfills even the extremely high requirements of coagulation tests. Simultaneously due to the fact that the test element temperature sensor need not provide absolute temperature values it can be incorporated relatively inexpensively into the disposable test elements. The instrument temperature sensor 15 needs not be located inside the instrument. Rather it can be even advantageous to locate sensor 15 at the outside of the instrument in a position where the environmental temperature is measured.

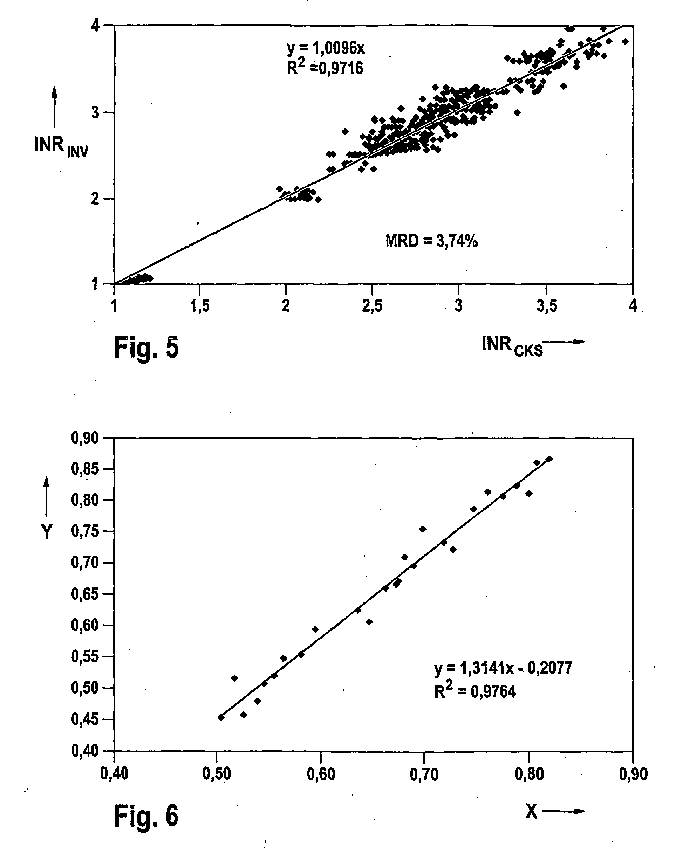

Figure 4 shows experimental results which were generated as follows:

- Samples from 27 different patients and four standard liquids ("normals were taken; each separated in multiple aliquots.

- From each sample four measurements of $PT^T$-values were made at four non-standard temperatures between 16° and 32°C (using four aliquots). These measurements were performed with a Coagu-Check®S instrument as manufactured by the applicant which was modified by disabling its thermostating system. Temperature variation was provided by positioning the instrument in a temperature chamber. _

- In order to improve the precision and increase the data base each of these measurements was performed with four different instruments.

- Simultaneously for each sample $PT^{37}$ was determined using a conventional CoaguCheck®S system with thermostating.

From the results PT-ratios $Y=PT^{37}/PT^T$ were calculated. These PT-ratios are shown in figure 2 relative to a temperature ratio X calculated by dividing the respective measurement temperature T (in °C) by 37.

Figure 4 shows that all data are very close to a curve $Y=f(X)$ determined by regression analysis therefrom. In this case the curve is a second order polynomial as shown in figure 4. This prooves the surprising fact that the temperature dependence can be described by a single functional relationship independent on the source of the samples used.

With respect to the temperature it was convenient during the practical experiments to use temperature ratios (as shown in place of the absolute temperatures). Evidently $Y=f(X)$ can be easily transformed to $Y=PT^{37}/PT^T=f(T)$. Furthermore an equation for calculating PT 37 is easily found by solving this equation for $PT^{37}$.

$$PT^{37} = f(T) * PT^T$$

Figure 5 shows a comparison of INR-values determined according to the invention (designated $INR_{INV}$) with corresponding data determined by a reference method (INR$_{CKS}$). INR values are calculated by forming a ratio between PT and a Median Normal PT (MNPT) as is well known in the art. The figure shows that the results generated by both methods are in excellent agreement with a Mean Relative Deviation (MRD) of 3,74%. It should be noted that the values of INR$_{INV}$ were determined with ambient temperature measurements between about 16°C and 32°C whereas the reference values INR$_{CKS}$ were determined at the standard temperature of 37°C.

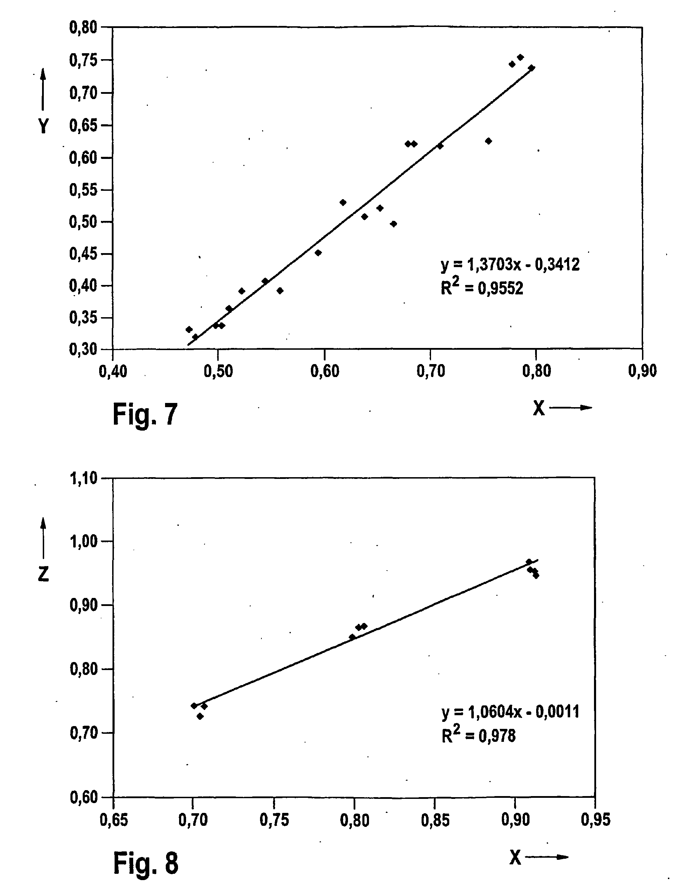

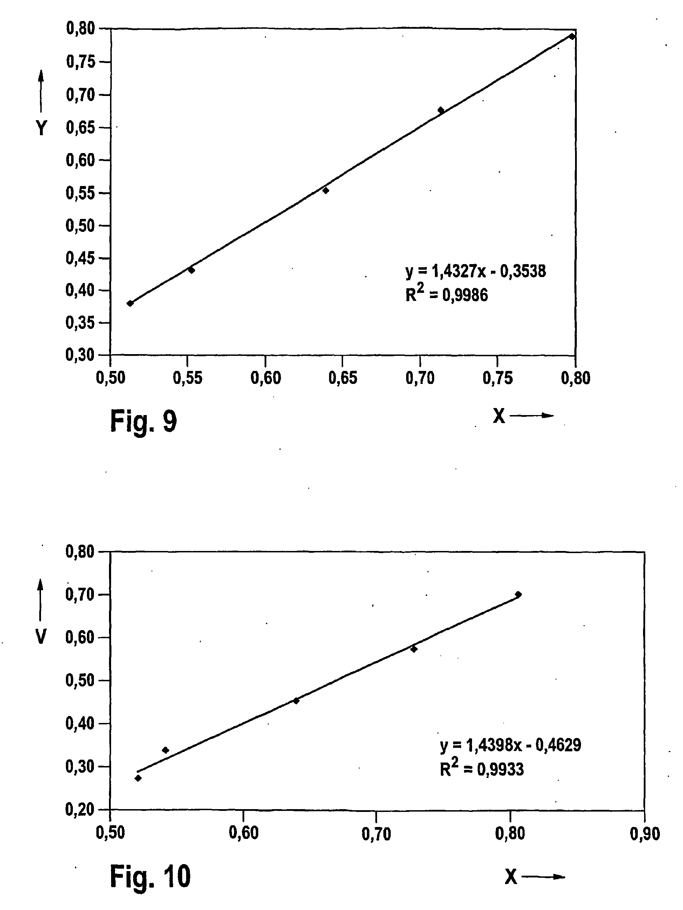

Figures 6 to 10 show the results of experiments performed with a smaller number of samples but a plurality of different methods. In each case a ratio of a coagulation parameter at a standard temperature and the same parameter at the respective non-standard temperature (designated Y for PT-ratios, Z for INR-ratios and V for APTT-ratios) is plotted against the temperature ratio X. The individual figures are based on experiments using the following coagulation test systems:

Figure 6:

CoaguCheck® S Low ISI PT Strips. These tests use dry Low ISI thromboplastin derived from human recombinant tissue factor requiring non-anticoagulated fresh venous of capillary blood. The samples were from patients on oral anticoagulant treatment.

Figure 7:

CoaguCheck® S Low Volume PT-Strips. These strips contain High ISI thromboplastin derived from rabbit brains requiring non-anticoagulated fresh venous or capillary blood.

Figure 8:

Amelung 4 Channel Lab Analyzer with Low ISI Lab reagent. This reagent contains Ortho Recombiplastin PT derived from human recombinant tissue factor requiring citrated plasma.

Figure 9:

Amelung 4 Channel Lab Analyzer with High ISI Lab reagent. This reagent includes Dade C Plus PT derived from rabbit brains requiring citrated plasma.

Figure 10:

Amelung 4 Channel Lab Analyzer with Ortho Auto APTT Lab reagent requiring citrated plasma.

In each case the resulting data can be described for all samples by a single functional relationship as shown in the figures. Thus the invention is applicable to different types of coagulation tests including both "dry chemistry" and "wet chemistry" test.

## Claims

1. System for the determination of a coagulation parameter of blood or plasma samples of patients, said system comprising

a,disposable test element (2) including a reagent system (38) to be mixed with the sample, thereby forming a coagulation detection liquid, and a coagulation detection zone (5) where the coagulation detection liquid is contained for monitoring a measurable property of the coagulation detection liquid, said measurable property changing during coagulation, and
an instrument (1) with a holder (8) for holding said disposable test element (2) inserted therein and with a measurement and evaluation electronics (13) adapted for detecting a signal which corresponds to the measurable property of the coagulation detection liquid and including a time measurement electronics for measuring a coagulation time required for said change to occur for deriving the desired coagulation parameter therefrom,
**characterized in that**

the system is adapted to determine the coagulation time at a non-standard temperature of the coagulation detection liquid different from a standard temperature for the respective coagulation parameter, and comprises a temperature measurement device (7) for measuring said non-standard temperature,
the instrument (1) has a non-volatile memory (18) containing data which define a mathematical relationship of coagulation time versus temperature which relationship is independent of the individual patient whose blood is examined, and
the system is adapted for calculating the coagulation parameter for the standard temperature from the coagulation time measured at said non-standard temperature, using said mathematical relationship.

2. System according to claim 1, wherein the instrument contains no device for thermostating the test element with the coagulation detection liquid contained therein, whereby the temperature of the test element and of the coagulation detection liquid depends on the temperature of the environment in which the measurement is performed.

3. System according to any one of the preceding claims, wherein the instrument is battery-operated.

4. System according to any one of the preceding claims, wherein

the disposable test element includes an element temperature sensor (14) integrated therein, the instrument includes an instrument temperature sensor (15) the measurement and evaluation electronics (13) is adapted to derive

- from the signal of the instrument temperature sensor (15)a temperature value relative to the standard temperature and .
- from the signal of the element temperature sensor (14)an information about the speed of change of the element temperature versus time and

the measurement and evaluation electronics (13) uses the temperature value derived from the instrument temperature sensor for calculating the coagulation parameter for said standard temperature from the coagulation time measured at said non-standard temperature only when the speed of change of the element temperature versus time is smaller than a limiting value thereof.

5. System according to any one of the precedings claims, wherein the coagulation detection zone (6) is outside the instrument housing when the test element (2) is inserted in the holder.

6. System according to any one of the preceding claims, wherein the instrument (1) comprises an interchangeable machine readable data storage element, in particular a ROM key (16), said interchangeable data storage element including the memory (18) containing the data which define the mathematical relationship of coagulation time versus temperature.

7. System according to claim 6 wherein the test element comprises a machine readable code (10) containing identification data which are specific for the manufacturing lot of the test element (2) and the instrument has a reader (11) for reading said machine readable identification code (10) for checking by the measurement and evaluation electronics (13) that a ROM key (16) inserted into the instrument corresponds to the manufacturing lot of the test element (2).

8. System according to claim 1, wherein the measurable property is the activity of an enzyme which participates in the coagulation pathway of the coagulation detection liquid.

9. System according to claim 8 wherein the system comprises electrochemical means for detecting the activity of the enzyme.

10. System according to claim 1, wherein the same mathematical relationship is used for all determinations of a given coagulation parameter performed with the instrument; independent of the reagent lot.

11. System according to claim 1, wherein the mathematical relationship is determined by measuring the coagulation time for a plurality of different samples at different temperatures using the same type of instrument and reagents.

12. Disposable test element adapted for a system according to any one of claims 1 to 11,

said test element including a reagent system (38) to be mixed with a sample, thereby forming a coagulation detection liquid, and a coagulation detection zone (5) where the coagulation detection liquid is contained for monitoring a measurable property.of the coagulation detection liquid, said measurable property changing during coagulation,
**characterized in that** it comprises a temperature measurement sensor (14), in particular a thermistor, and plug-in contacts allowing transfer of temperature signals of the temperature sensor to an instrument into which it is inserted.

13. Method for the determination of a coagulation parameter of a blood or plasma sample of a patient, wherein

a reagent system (38) is mixed in a disposable test element (2) with the sample to form a coagulation detection liquid in a reaction detection zone (6) of the element (2) and a measurable property of the coagulation detection liquid is monitored, said measurable property changing during coagulation,
a signal which corresponds to the measurable property of the coagulation detection liquid is detected and a time required for said change to occur is measured for deriving the desired coagulation parameter therefrom,
**characterized in that**

the coagulation time is determined at a nonstandard temperature of the coagulation detection liquid different from a standard temperature for the respective coagulation parameter, said non-standard temperature being measured,
data which define a mathematical relationship of coagulation time versus temperature are taken from a non-volatile memory (18) of the instrument (1), said relationship being independent of the individual patient whose blood is examined, and

the coagulation parameter for the standard temperature is calculated from the coagulation time measured at said non-standard temperature, using a mathematical relationship of coagulation time versus temperature which relationship is independent of the individual patient whose blood is examined.

14. Method according to claim 13, wherein the measurable property is the activity of an enzyme which participates in the coagulation pathway of the coagulation detection liquid.

15. Method according to claim 14 wherein the activity of the enzyme is detected by electrochemical means.

16. Method according to claim 13, wherein the same mathematical relationship is used for all determinations of a given coagulation parameter performed with the instrument, independent of the reagent lot.

17. Method according to claim 13, wherein the mathematical relationship is determined by measuring the coagulation time for a plurality of different samples at different temperatures using the same type of instrument and reagents.

**Patentansprüche**

1. System zur Bestimmung eines Koagulationsparameters in Blut- oder Plasmaproben von Patienten, umfassend

ein disposibles Testelement (2), welches ein Reagenzsystem (38) zur Mischung mit der Probe unter Bildung einer Koagulationsdetektionsflüssigkeit enthält und welches eine Koagulationsdetektionszone (5) aufweist, in die die Koagulationsdetektionsflüssigkeit zur messtechnischen Beobachtung einer messbaren Eigenschaft der Koagulationsdetektionsflüssigkeit aufgenommen wird, wobei sich die messbare Eigenschaft während der Koagulation ändert, und

ein Instrument,(1) mit einem Halter (8) zum Halten eines darin eingesetzten disposiblen Testelementes (2) und mit einer Mess- und Auswerteelektronik (13), die dazu ausgebildet ist, ein Signal zu detektieren, welches der messbaren Eigenschaft der Koagulationsdetektionsflüssigkeit entspricht und die eine Zeitmesselektronik einschließt, um, eine Koagulationszeit bis zum Auftreten der Änderung der messbaren Eigenschaft zu messen und daraus den gewünschten Koagulationsparameter abzuleiten, **dadurch gekennzeichnet, dass**

das System dazu ausgebildet ist, die Koagulationszeit der Koagulationsdetektionsflüssigkeit bei einer Nicht-Standard-Temperatur zu messen, welche sich von einer Standard-Temperatur des betreffenden Koagulationsparameters unterscheidet und das System eine Temperaturmesseinrichtung (7) zur Messung der Nicht-Standard-Temperatur aufweist,

das Instrument (1) einen nichtflüchtigen Speicher (18) aufweist, welcher Daten enthält, die eine mathematische Beziehung zwischen der Koagulationszeit und der Temperatur definieren, welche Beziehung unabhängig von dem individuellen Patienten ist, dessen Blut untersucht wird, und

das System dazu ausgebildet ist, den Koagulationsparameter für die Standard-Temperatur aus der Koagulationszeit, die bei der Nicht-Standard-Temperatur gemessen wurde, unter Verwendung der mathematischen Beziehung zu berechnen.

2. System nach Anspruch 1, bei welchem das Instrument keine Einrichtung zur Thermostatisierung des Testelements mit der darin enthaltenen Koagulationsdetektionsflüssigkeit aufweist, so dass die Temperatur des Testelementes und der Koagulationsdetektionsflüssigkeit von der Temperatur der Umgebung abhängt, in der die Messung durchgeführt wird.

3. System nach einem der vorhergehenden Ansprüche, **dadurch gekennzeichnet, dass** das Instrument batteriebetrieben ist.

4. System nach einem der vorhergehenden Ansprüche, bei welchem

das disposible Testelement einen integrierten Element-Temperatursensor (14) aufweist, das Instrument einen Instrument-Temperatursensor (15) aufweist, die Mess- und Auswerteelektronik (13) dazu ausgebildet ist,

- aus dem Signal des Instrument-Temperatursensors (15) einen Temperaturwert relativ zu der Standard-Temperatur abzuleiten und .
- aus dem Signal des Element-Temperatursensors (14) eine Information über die Geschwindigkeit der Änderung der Elementtemperatur gegen die Zeit abzuleiten, und '
- die Mess- und Auswerteelektronik (13) die mittels des Instrument-Temperatursensors ermittelte Temperatur nur dann zur Berechnung des Koagulationsparameters für die Standard-Temperatur aus der bei der Nicht-Standard-Temperatur gemessenen Koagulationszeit verwendet, wenn die Änderungsgeschwindigkeit der Elementtemperatur je Zeiteinheit geringer als ein diesbezüglicher Grenzwert ist.

5. System nach einem der vorhergehenden Ansprüche, bei welchem sich die Koagulationsdetektionszone (6) außerhalb des Gehäuses des Instrumentes befindet, wenn das Testelement (2) in den Halter eingesetzt ist.

6. System nach einem der vorhergehenden Ansprüche, bei welchem das Instrument (1) ein auswechselbares maschinenlesbares Datenspeicherelement, insbesondere einen ROM-Baustein (16), einschließt, wobei das auswechselbare Datenspeicherelement den Speicher (18) einschließt, welcher die Daten enthält, die die mathematische Beziehung zwischen der Koagulationszeit und der Temperatur definieren.

7. System nach Anspruch 6, bei welchem das Testelement einen maschinenlesbaren Code aufweist, welcher Identifikationsdaten enthält, die für die Herstellungscharge des Testelements (2) spezifisch sind und bei welchem das Instrument einen Leser (11) zum Lesen des maschinenlesbaren Identifikationscodes (10) aufweist, um mittels der Mess- und Auswerteelektronik (13) zu überprüfen, dass ein in das Instrument eingesetzter ROM-Baustein (16) mit der Herstellungscharge des Testelementes (2) korrespondiert.

8. System nach Anspruch 1, bei welchem die messbare Eigenschaft die Aktivität eines Enzyms ist, das an dem Koagulationspfad der Koagulationsdetektionsflüssigkeit teilnimmt.

9. System nach Anspruch 8, welches elektrochemische Mittel zur Detektion der Aktivität des Enzyms einschließt.

10. System nach Anspruch 1, bei welchem die gleiche mathematische Beziehung für alle mit dem Instrument durchgeführten Bestimmungen eines vorgegebenen Koagulationsparameters, unabhängig von der Reagenzcharge, verwendet wird.

11. System nach Anspruch 1, bei welchem die mathematische Beziehung dadurch bestimmt wird, dass man die Koagulationszeit für eine Mehrzahl von verschiedenen Proben bei verschiedenen Temperaturen bestimmt, wobei der gleiche Instrumententyp und der gleiche Reagenztyp verwendet werden.

12. Disposibles Testelement, welches als Bestandteil eines Systems nach einem der Ansprüche 1 bis 11 ausgebildet ist, wobei das Testelement ein Reagenzsystem (38) zur Mischung mit der Probe unter Bildung einer Koagulationsdetektionsflüssigkeit enthält und eine Koagulationsdetektionszone (5) aufweist, in die die Koagulationsdetektionsflüssigkeit zur messtechnischen Beobachtung einer messbaren Eigenschaft der Koagulationsdetektionsflüssigkeit aufgenommen wird, wobei sich die messbare Eigenschaft während der Koagulation ändert, **dadurch gekennzeichnet, dass** es einen Temperaturmesssensor (14), insbesondere einen Thermistor, aufweist, sowie Steckkontakte, die die Übertragung von Temperatursignalen des Temperatursensors an ein Instrument, in das er eingesetzt ist, ermöglichen.

13. Verfahren zur Bestimmung eines Koagulationsparameters in einer Blut- oder Plasmaprobe eines Patienten, bei welchem

ein Reagenzsystem (38) in einem disposiblen Testelement (2) mit der Probe gemischt wird, um eine Koagulationsdetektionsflüssigkeit in einer Reaktionsdetektionszone (6) des Testelementes (2) zu bilden und eine messbare Eigenschaft der Koagulationsdetektionsflüssigkeit messtechnisch beobachtet wird, wobei sich die messbare Eigenschaft während der Koagulation ändert,

ein Signal, das mit der messbaren Eigenschaft der Koagulationsdetektionsflüssigkeit korrespondiert, detektiert wird und eine Zeit gemessen wird, die vergeht, bis die Änderung auftritt, um den gewünschten Koagulationsparameter daraus abzuleiten,

**dadurch gekennzeichnet, dass**

die Koagulationszeit bei einer Nicht-Standard-Temperatur der Koagulationsdetektionsflüssigkeit bestimmt wird, die sich von einer Standard-Temperatur für den betreffenden Koagulationsparameter unterscheidet, wobei die Nicht-Standard-Temperatur gemessen wird,

Daten, die eine mathematische Beziehung zwischen der Koagulationszeit und der Temperatur definieren, aus einem nicht-flüchtigen Speicher (18) des Instrumentes (1) ausgelesen werden, wobei die Beziehung unabhängig von dem individuellen Patienten ist, dessen Blut untersucht wird, und

der Koagulationsparameter für die Standard-Temperatur aus der bei einer Nicht-Standard-Temperatur gemessenen Koagulationszeit berechnet wird, wobei eine mathematische Beziehung zwischen der Koagulationszeit und der Temperatur verwendet wird, welche unabhängig von dem individuellen Patienten ist, dessen Blut untersucht wird.

**14.** Verfahren nach Anspruch 13, bei welchem die messbare Eigenschaft die Aktivität eines Enzyms ist, das an dem Koagulationspfad der Koagulatiönsdetektionsflüssigkeit teilnimmt.

**15.** Verfahren nach Anspruch 14, bei welchem die Aktivität des Enzyms mit elektrochemischen Mitteln detektiert wird.

**16.** Verfahren nach Anspruch 13, bei welchem die gleiche mathematische Beziehung für alle Bestimmungen eines gegebenen Koagulationsparameters verwendet wird, die mit dem Instrument durchgeführt werden, unabhängig von der Reagenzcharge.

**17.** Verfahren nach Anspruch 13, bei welchem die mathematische Beziehung dadurch bestimmt wird, dass man die Koagulationszeit für eine Mehrzahl von verschiedenen Proben bei verschiedenen Temperaturen bestimmt, wobei der gleiche Instrumententyp und der gleiche Reagenztyp verwendet wird.

**Revendications**

**1.** Système pour déterminer un paramètre de coagulation d'échantillons de sang ou de plasma de patients, ledit système comprenant :

■ un élément de test jetable (2) comprenant un système de réactifs (38) destiné à être mélangé avec l'échantillon, formant ainsi un liquide de détection de la coagulation, et une zone de détection de la coagulation (5) contenant le liquide de détection de la coagulation, destinée, à surveiller une propriété mesurable du liquide de détection de la coagulation, ladite propriété mesurable changeant durant la coagulation, et
■ un instrument (1) comprenant un support (8) destiné à maintenir ledit élément de test jetable (2) inséré dans ledit support, ainsi qu'un moyen électronique (13) de mesure et d'évaluation conçu pour détecter un signal correspondant à la propriété mesurable du liquide de détection de la coagulation, et comprenant un moyen électronique de mesure du temps destiné à mesurer un temps de coagulation requis pour la réalisation dudit changement, à partir duquel le paramètre voulu de coagulation peut être dérivé,

**caractérisé en ce que**

■ le système est conçu pour déterminer le temps de coagulation à une température non standard du liquide de détection de la coagulation, différente d'une température standard pour le paramètre de coagulation respectif et comprend un dispositif de mesure de la température (7), destiné à mesurer ladite température non standard,
■ l'instrument (1) dispose d'une mémoire non volatile (18) contenant des données définissant une relation mathématique du temps de coagulation en fonction de la température, ladite relation étant indépendante du patient individuel dont le sang est examiné, et
■ le système est conçu pour calculer le paramètre de coagulation pour la température standard à partir, du temps de coagulation mesuré à ladite température non standard, en utilisant ladite relation mathématique.

**2.** Système selon la revendication 1, dans lequel l'instrument ne contient pas de dispositif destiné à thermostater l'élément de test contenant le liquide de détection de la coagulation, la température de l'élément de test et du liquide de détection de la coagulation dépendant de la température de l'environnement dans lequel la mesure est réalisée.

**3.** Système selon l'une quelconque des revendications précédentes, dans lequel, l'instrument fonctionne avec une batterie.

**4.** Système selon l'une quelconque des revendications précédentes, dans lequel

■ l'élément de test jetable comprend un capteur de la température de l'élément (14) intégré au dit élément,
■ l'instrument comprend un capteur de la température de l'instrument (15)
■ le moyen électronique de mesure et d'évaluation (13) est conçu pour dériver

■ du signal du capteur de la température de l'instrument (15) une valeur de température relative à la température standard et
■ du signal du capteur de la température de l'élément (14) une information relative à la vitesse de changement de la température de l'élément en fonction du temps et
■ le moyen électronique de mesure et d'évaluation (13) utilise la valeur de température dérivée du capteur de la température de l'instrument pour calculer le paramètre de coagulation pour ladite température standard à partir du temps de coagulation mesuré à ladite température non standard uniquement lorsque la vitesse de changement de la température de l'élément en fonction du temps est inférieure à une valeur limite.

5. Système selon l'une quelconque des revendications précédentes, dans lequel la zone de détection de la coagulation (6) est située à l'extérieur du boîtier de l'instrument lorsque l'élément de test (2) est inséré dans le support.

6. Système selon l'une quelconque des revendications précédentes, dans lequel l'instrument (1) comprend un élément interchangeable de stockage de données, lisible par machine notamment une clé ROM (16), ledit élément interchangeable de stockage des données comprenant la mémoire (18) contenant les données définissant la relation mathématique du temps de coagulation en fonction de la température.

7. Système selon la revendication 6, dans lequel l'élément de test comprend un code lisible par machine (10) contenant des données d'identification spécifiques du lot de fabrication de l'élément de test (2) et l'instrument possède un lecteur (11) destiné à lire ledit code d'identification lisible par machine (10) afin de vérifier par le moyen électronique de mesure et d'évaluation (13) qu'une clé ROM (16) insérée dans l'instrument correspond au lot de fabrication de l'élément de test (2).

8. Système selon la revendication 1, dans lequel la propriété mesurable est l'activité d'une enzyme qui participe à la voie de coagulation du liquide de détection de la coagulation.

9. Système selon la revendication 8, dans lequel le système comprend des moyens électrochimiques de détection de l'activité de l'enzyme.

10. Système selon la revendication 1, dans lequel la même relation mathématique est utilisée pour toutes les déterminations d'un paramètre de coagulation donné réalisées avec l'instrument, indépendamment du lot de réactifs.

11. Système selon la revendication 1, dans lequel la relation mathématique est déterminée en mesurant le temps de coagulation pour une pluralité d'échantillons différents à différentes températures en utilisant le même type d'instrument et de réactifs.

12. Élément de test jetable conçu pour un système selon l'une quelconque des revendications 1 à 11, ledit élément de test comprenant un système de réactifs (38) destiné à être mélangé avec un échantillon, formant ainsi un liquide de détection de la coagulation, et une zone de détection de la coagulation (5) contenant le liquide de détection de la coagulation, destinée à surveiller une propriété mesurable du liquide de détection de la coagulation, ladite propriété mesurable changeant durant la coagulation, **caractérisé en ce qu'**il comprend un capteur de mesure de la température (14), notamment une thermistance, et des contacts enfichables permettant le transfert de signaux de température du capteur de température vers un instrument dans lequel il est inséré.

13. Procédé de détermination d'un paramètre de coagulation d'un échantillon de sang ou de plasma d'un patient, dans lequel

   ■ un système de réactifs (38) est mélangé dans un élément de test jetable (2) avec l'échantillon pour former un liquide de ' détection de la coagulation dans une zone de détection d'une réaction (6) de l'élément (2) et une propriété mesurable du liquide de détection de la coagulation est surveillée, ladite propriété mesurable changeant durant la coagulation,
   ■ un signal correspondant à la propriété mesurable du liquide de détection de la coagulation est détecté et un temps requis pour la réalisation dudit changement est mesuré pour en dériver ledit paramètre voulu de coagulation,

   **caractérisé en ce que**

   ■ le temps de coagulation est déterminé à une température non standard du liquide de détection de la coagulation, différente d'une température standard pour le paramètre de coagulation respectif, ladite température non standard étant mesurée,
   ■ des données définissant une relation mathématique du temps de coagulation en fonction de la température sont prises à partir d'une mémoire non volatile (18) de l'instrument (1), ladite relation étant indépendante du patient individuel dont le sang est examiné, et
   ■ le paramètre de coagulation pour la température standard est calculé à partir du temps de coagulation mesuré à ladite température non standard, en utilisant une relation mathématique du temps de coagulation en fonction de la température, ladite relation étant indépendante du patient individuel dont le sang est examiné.

14. Procédé selon la revendication 13, dans lequel la propriété mesurable est l'activité d'une enzyme qui participe à la voie de coagulation du liquide de détection de la coagulation.

15. Procédé selon la revendication 14, dans lequel l'activité de l'enzyme est détectée par des moyens électrochimiques.

16. Procédé selon la revendication 13, dans lequel la même relation mathématique est utilisée pour toutes les déterminations d'un paramètre de coagulation donné réalisées avec l'instrument, indépendamment du lot de réactifs.

17. Procédé selon la revendication 13, dans lequel la relation mathématique est déterminée en mesurant le temps de coagulation pour une pluralité d'échantillons différents à différentes températures ,en utilisant le même type d'instrument et de réactifs.

**Fig. 1**

$$y = 0.514x^2 + 0.5235x + 0.0129$$
$$R^2 = 0.969$$

$$Y = \frac{PT^{37}}{PT^0}$$

$$Y = f(X)$$

**Fig. 4**

$$X = \frac{T\ [°C]}{37}$$

Fig. 2

Fig. 3

EP 1 588 161 B1

Fig. 5

Fig. 6

17

Fig. 7

$$y = 1{,}3703x - 0{,}3412$$
$$R^2 = 0{,}9552$$

Fig. 8

$$y = 1{,}0604x - 0{,}0011$$
$$R^2 = 0{,}978$$

**Fig. 9**

$y = 1{,}4327x - 0{,}3538$
$R^2 = 0{,}9986$

**Fig. 10**

$y = 1{,}4398x - 0{,}4629$
$R^2 = 0{,}9933$

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5789664 A
- WO 0111356 A
- WO 0163271 A
- US 5580744 A

- US 5405511 A
- US 5972715 A
- US 5031619 A

**Non-patent literature cited in the description**

- **ULDALL.** Prothrombin time standardization and temperature problems. *Clinica Chimica Acta,* 1980, 39-44